# EUROPEAN PATENT APPLICATION

(11) **EP 4 464 275 A1**
(43) Date of publication of application: **20.11.2024**
(21) Application number: 23305782.7
(22) Date of filing: 16.05.2023
(51) Int. Cl.: A61D 19/02, A61B 17/43

(54) **INSEMINATION WORK TOOL AND WORK ASSEMBLY COMPRISING THIS TOOL AND AN APPARATUS FOR ASSISTING IN VAGINAL PENETRATION**

(71) Applicant: IMV Technologies, 61300 Saint-Ouen-sur-Iton (FR)
(72) Inventor: SCHMITT, Eric, 53700 Villaines-la-Juhel (FR); CHEVRIER, Ludivine, 61270 Beaufai (FR)
(74) Representative: Santarelli

(57) **Abstract**

The insemination work tool comprises an insemination gun (32), which is to be used with a straw for the preservation of a predetermined dose of liquid-based substance and in particular pure or diluted animal semen, and a gun extension (145).

The work assembly comprises this insemination work tool and an apparatus for assisting in vaginal penetration, in particular for farm animals, provided to receive the insemination work tool.

## Description

### FIELD OF THE INVENTION

The invention relates to insemination guns to be used with a straw for the preservation of a predetermined dose of liquid-based substance, in particular pure or diluted animal semen, and more precisely to an insemination work tool comprising such an insemination gun as well as a gun extension.

The invention further relates to a work assembly comprising such an insemination work tool and an apparatus for assisting in vaginal penetration, in particular for farm animals, provided to receive the insemination work tool.

### TECHNOLOGICAL BACKGROUND

From PCT application WO 2016/066962, to which corresponds U.S. patent application US 2017/0319317, there is already known an apparatus for assisting in vaginal penetration which is provided to receive a work tool, comprising a handling shaft and a speculum tube extending axially from the handling shaft to a distal end at which it has an opening. The apparatus further comprises a video viewing system comprising an objective disposed within the speculum tube and a transmission device connected to the objective and connectable to a remotely located screen for viewing the image obtained by the objective. The apparatus also comprises an objective support to hold the objective in a predetermined position relative to the speculum tube in which the objective is in the neighborhood of the opening of the speculum tube and faces the space located beyond that opening.

The handling shaft is furthermore configured to slidingly guide the work tool in the speculum tube such that the work tool can reach an advanced position in which part of the work tool projects beyond the opening of the speculum tube.

To perform an artificial insemination, the work tool is an insemination gun equipped which a straw filled with semen. The speculum tube is inserted into the animal's vagina and once the apparatus is correctly positioned, the tool is pushed towards the advanced position in order for the tip of the sheath of the gun to be inserted through the uterine cervix, then the semen is ejected into the uterus.

Previously, the apparatus (and more specifically the transmission device of the video viewing system) is connected to a remotely located screen such as a smartphone screen. During the operation, the operator is able to observe on the remotely located screen the progression of the speculum tube, then of the insemination gun, thanks to the video viewing system which in real-time sends back the image of the space located beyond the opening of the speculum tube.

International application WO 2019/122685, to which corresponds U.S. patent application US 2021/0085169, discloses such an apparatus provided to receive a work tool comprising the insemination gun as well as a gun extension.

A particular embodiment of a work assembly comprising this apparatus and this work tool is described in details below with reference to Figures 1 to 5 of the accompanying drawings. In these:
- Figure 1 is a side view of this work assembly in an initial configuration in which the work tool is in a withdrawn position on the handling shaft of the apparatus, in which the end of the work tool is located inside the speculum tube of the apparatus;
- Figure 2 is a diagrammatic view in longitudinal cross-section of a straw for the preservation of a predetermined dose of liquid-based substance, in particular pure or diluted animal semen, suitable for being used with the work tool;
- Figure 3 is a longitudinal section view of the end portion of the assembly which can be seen on the right in Figure 1, showing the work tool with the preservation straw of Figure 2 received inside, the video viewing system and a support block configured to slidingly guide the work tool and hold the objective of the video viewing system in a predetermined position;
- Figure 4 is a similar view to Figure 1 but showing the assembly in an intermediate configuration in which the work tool is in an advanced position on the handling shaft of the apparatus, in which the end of the work tool projects forward from the speculum tube; and
- Figure 5 is a similar view to Figure 4 but showing the assembly in a final configuration in which the semen has been ejected out from the straw and from the work tool.

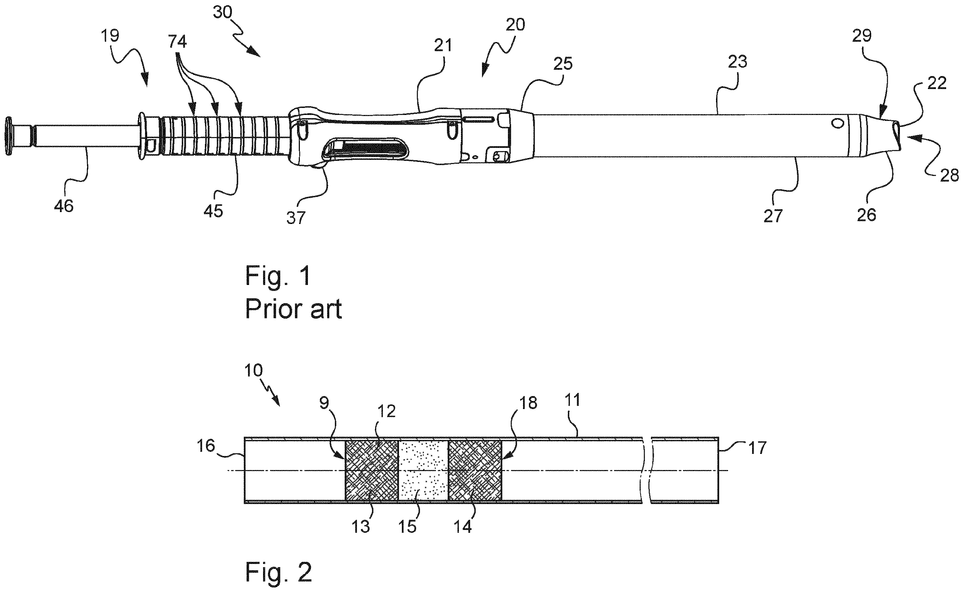

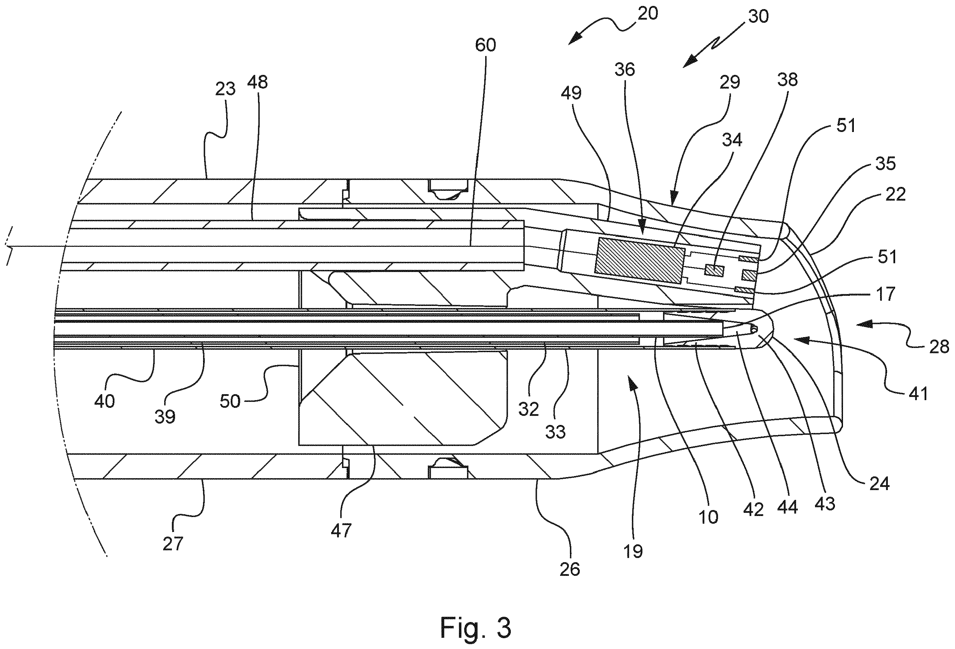

Figures 1 to 3 illustrate a work assembly 30 comprising an apparatus 20 for assisting in vaginal penetration, a work tool 19 slidingly received in the apparatus 20 and a straw 10 (Figures 2 and 3) for preservation of a predetermined dose of liquid-based substance, here pure or diluted animal semen which is used with the work tool 19.

The work tool 19 is used here for the artificial insemination of farm animals with the semen contained in the straw 10.

As explained below, the apparatus 20 makes it possible to facilitate the insertion of the work tool 19 into the vagina of the animal and the guiding of the work tool 19 to the uterine cervix, beyond which the semen is to be injected.

The apparatus 20 and the work tool 19 are configured here for cattle.

The apparatus 20 comprises a handling shaft 21 and a speculum tube 23 extending axially from the handling shaft 21 to a distal end 22 at which the speculum tube 23 has an opening 28.

The handling shaft 21 is formed by a tubular body open at both ends.

The speculum tube 23 is coaxial here to the handling shaft 21.

The apparatus 20 thus has an elongate generally cylindrical shape.

The speculum tube 23 is fastened to the handling shaft 21 via a fastening ring 25.

The ring 25 is fastened in the neighborhood of the proximal end of the speculum tube 23 (the end oriented towards the handling shaft 21), here by bonding.

The fastening ring 25 is attached to the distal end of the handling shaft 21 (the end oriented towards the speculum tube 23), here by snap engagement.

The ring 25 and the handling shaft 21 are configured so that the ring 25 can have its snap engagement with the handling shaft 21 undone, the speculum tube 23 thus being removable.

The speculum tube 23 comprises an end part 26, delimiting the opening 28, and a main part 27 extending between the end part 26 and the handling shaft 21.

In other words, the end part 26 extends between the main part 27 and the distal end 22 of the speculum tube 23 which is also the distal end of the end part 26.

The opening 28 is delimited by the edge of the end part 26 located at the distal end 22.

The end part 26 is of elastically deformable material, whereas the main part 27 is of rigid material.

The material of the end part 26 here comprises a thermoplastic elastomer material (or TPE) which has a hardness of about 40 Shore D. Generally, the material of the end part 26 has a hardness comprised between 35 and 45 Shore D.

The material of the end part 26 has an elastic limit extension which is approximately 20% here. Generally, this elastic limit extension can be equal to or greater than approximately 20%.

The end part 26 is formed here as a single part.

The material of the main part 27 here comprises PMMA, which is a transparent rigid thermoplastic material.

It will be noted that the end part 26 has an outside surface 29 of which the diameter increases between the opening 28 and the main part 27.

More specifically, this diameter increases until it reaches the outside diameter of the main part 27 which itself remains constant along the length of the main part 27.

Furthermore, the outside surface 29 increases in diameter such that it has a concave curved profile of which the concavity is oriented towards the outside of the tube 23.

The apparatus 20 further comprises a video viewing system comprising an objective 35 (Figure 3) disposed within the speculum tube 23 and a transmission device 36 connected to the objective 35 and connectable to a remotely located screen (not illustrated) for viewing the image obtained by the objective 35.

The transmission device 36 comprises an electronic circuit 34, a photosensor 38 connected to the electronic circuit 34, a plug socket 37 (Figure 1) and a cable 60 connecting the electronic circuit 34 to the plug socket 37.

The photosensor 38 is disposed behind the objective 35 to receive the image obtained by the latter and is configured to convert that image into a signal which is then sent to the electronic circuit 34. The photosensor 38 is of CCD type here.

The cable 60 is configured to convey the signal from the electronic circuit 34 to the plug socket 37 which is housed in the wall of the handling shaft 21, to the rear of the latter (that is to say in the neighborhood of the opposite end of the handling shaft to the speculum tube).

The video viewing system further comprises one or more lighting members 51, here LED-based lighting members, connected to the electronic circuit 34. The lighting members 51 are juxtaposed to the objective 35 and disposed on opposite sides thereof.

The cable 60 and the plug socket 37 are each configured here both to convey the signal and the energy required for the operation of the electronic circuit 34, of the sensor 38 and of the lighting members 51.

The plug socket 37 is a connector of micro-USB type here.

To simplify the drawings, the electronic circuit 34, the sensor 38, the cable 60, the lighting members 51 and the objective 35 are represented very diagrammatically in Figure 3.

The remotely located screen here forms part of a smartphone (not illustrated) which is configured to be connected to the transmission device 36 using a suitable cable (not illustrated) connected both to the smartphone and to the plug socket 37.

The apparatus 20 further comprises a support block 47 and a mounting bar 48 each received in the speculum tube 23.

The bar 48 is rigidly fastened by each of its ends respectively to the handling shaft 21 and to the support block 47, the latter thus occupying a predetermined fixed position in the speculum tube 23.

The bar 48 is tubular here and receives the cable 60. On exiting the bar 48, towards the handling shaft 21, the cable 60 passes through the wall of the handling shaft 21 to reach the plug socket 37.

The support block 47 comprises an objective support 49 and a guiding support 50 for the work tool 19, which here form a single part.

The objective support 49 holds the objective 35 in a predetermined position relative to the speculum tube 23 in which the objective 35 is in the neighborhood of the opening 28 of the speculum tube and faces the space located beyond that opening 28, that is to say the space that extends in line with the tube 23.

The objective support 49 furthermore holds the lighting members 51, which are configured to illuminate the space located beyond the opening 28.

It will be noted that the end part 26 of the speculum tube 23 is disposed around the objective support 49.

The guiding support 50 is configured to guide the sliding of the part of the work tool 19 that is received in the speculum tube 23. The guiding support 50 is furthermore configured in order for the work tool 19 to slidingly move in an axial direction centered relative to the outside surface of the speculum tube 23.

It will be noted that the end part 26 of the speculum tube 23 is also disposed around the guiding support 50.

Generally, the end part 26 of the speculum tube 23 is disposed around the support block 47.

The work tool 19 here comprises an extension 45, a piston 46 slidingly mounted in the extension 45, a re-usable insemination gun 32 and a single-use sanitary sheath 33 (partially illustrated in Figure 3) which is used with the straw 10 (Figures 2 and 3).

The extension 45 is configured to be slidingly mounted in the handling shaft 21 and is provided on its outside surface with a plurality of annular grooves 74 regularly spaced with a predetermined pitch. The annular grooves 74 are configured to cooperate with a bead (not illustrated) provided on the inside surface of the handling shaft 21 so as to form detent stops for positioning the work tool 19 relative to the apparatus 20. The predetermined pitch is approximately 1 cm here.

The work tool 19 is received in the apparatus 20 with the extension 45 which is slidingly mounted in the handling shaft 21, while the part of the work tool 19 that is received in the speculum tube 23 is able to slide through the guiding support 50.

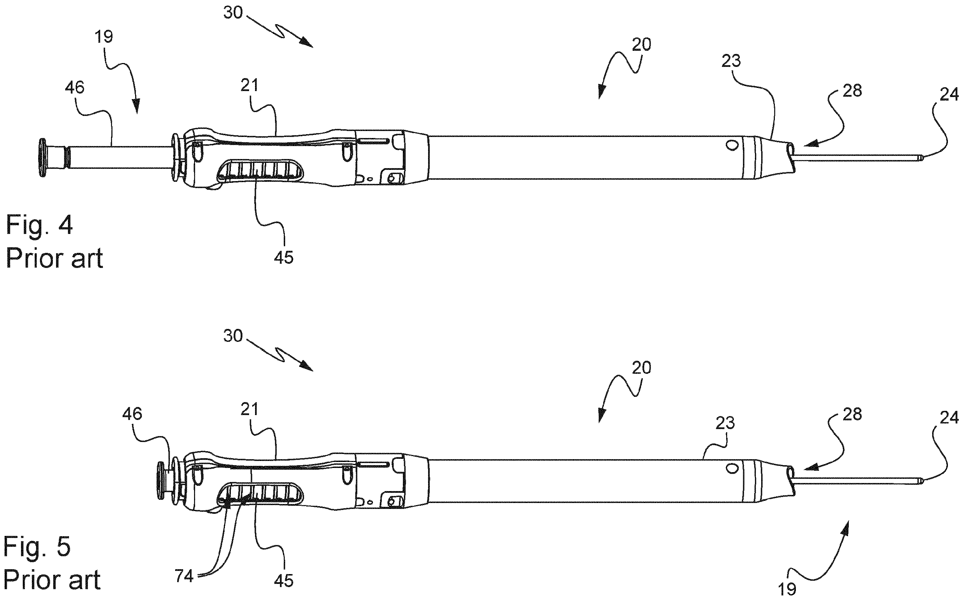

The work tool 19 is thus mounted movably in terms of translation relative to the apparatus 20 between a position that is withdrawn (Figures 1 and 3) relative to the apparatus 20, in which a distal end 24 of the work tool 19 is located inside the speculum tube 23 (that is to say inside the internal space of the tube 23 delimited by its wall), and a position that is advanced relative to the apparatus 20 (Figures 4 and 5), in which the distal end 24 of the tool 19 projects forwardly of the speculum tube 23.

In the withdrawn position, the distal end 24 of the tool 19 is thus situated on the same side of the opening 28 as the tube 23, that is to say rearward of the opening 28.

In the advanced position, the distal end 24 of the tool 19 is thus situated on the opposite side of the opening 28 from the tube 23, that is to say forward of the opening 28.

In Figure 3, the straw 10 can be seen received in the work tool 19. This straw 10 will now be described in more detail with reference to Figure 2.

The straw 10 illustrated in Figure 2 comprises a tube 11 and a stopper 12.

The tube 11 is conventionally made from extruded plastic material, with an inside diameter for example of 1.6 or 2.5 mm and a length of the order of 133 mm.

The stopper 12 is usually of the three-part type originally described in French patent 995.878, corresponding to British patent 669,265, i.e. formed by two plugs 13 and 14 made from a fibrous substance enclosing a powder 15 which, on contact with a liquid, is capable of transforming into an impermeable paste or gel adhering to the wall of the tube so that the stopper is liquid-tight.

In the initial state, shown in Figure 2, the stopper 12 is disposed in the neighborhood of the end 16 of the tube 11 and it is provided that in the filled state, the dose of liquid substance which must be preserved in the straw 10 is disposed between the stopper 12 and the end 17 of the tube 11 that is the furthest from the stopper 12.

In order to fill the straw 10, the end 16 is placed in communication with a vacuum source while the end 17 is placed in communication with a vessel containing the substance to be introduced into the straw.

The air initially contained between the stopper 12 and the end 17 is sucked through the stopper while the substance moves forward in the tube 11 until it encounters the stopper 12, by the end 18 thereof that is turned towards the end 17 of the tube 11, that is to say the end of the stopper 12 that can be seen on the right in Figure 2.

If necessary, the straw is welded in the neighborhood of one or both of its two ends 16 or 17 and is placed in cold storage.

To empty the straw 10, if necessary after cutting the welded end portions and thawing, there is inserted into the tube 11 a rod which comes to bear on the end 9 of the stopper 12 (which end is situated on the opposite side to the end 18). Using this rod, the stopper 12 is made to slide in the manner of a piston towards the end 17 or the end which corresponds after cutting the welded portion, which causes the expulsion of the dose of substance which had been introduced into the straw.

A description will now be given in more detail of the work tool 19 and of its cooperation with the straw 10.

The insemination gun 32 (Figure 3) of the work tool 19 comprises a rigid tubular body 39, for receiving the straw 10 filled with semen, and comprises the rod for driving the stopper 12, slidingly mounted in the rigid tubular body 39.

The extension 45 of the work tool 19 is mechanically connected to the rigid tubular body 39 of the gun 32, while the piston 46 is mechanically connected to the rod of the gun 32.

The sliding of the piston 46 relative to the extension 45 thus drives the sliding of the rod relative to the rigid tubular body 39 of the gun 32.

Prior to insertion of the straw 10 into the rigid tubular body 39, the inseminator takes the piston 46 as far as possible out from the extension 45, in order for the rod to be out or withdrawn as far as possible from the body 39 at the proximal end, that is to say at the end which is manipulated by the inseminator during the operation, then the straw 10 is inserted into the rigid tubular body 39 at its distal end (the far end from the proximal end), the straw 10 being inserted with the end 16 of the tube 11 (the end closest to the stopper 12), first. The straw 10 is pushed into the rigid tubular body 39 of the gun 32 until the end 16 of the tube 11 encounters a shoulder forming a pushing-in stop.

The straw 10 is then in place in the rigid tubular body 39 of the gun 32. The end 17 of the tube 11 as well as a certain length of the tube 11 starting from that end remain outside the rigid tubular body 39, that is to say that a certain part of the straw 10 projects beyond the distal end of the rigid tubular body 39 of the gun 32.

The sanitary sheath 33 (Figure 3) comprises a tube 40 of which the inside diameter is such that the rigid tubular body 39 of the gun 32 may be inserted therein. At one end (the proximal end) the tube 40 of the sanitary sheath 33 is open and at the other end (distal end) the sanitary sheath 33 comprises a tip 41 mechanically connected to the tube 40.

This tip 41 comprises a tail 42 inserted into an end portion of the tube 40 and a head 43 disposed in line with the tube 40.

The tip 41 comprises a duct 44 opening into the tube 40 at the proximal end of the tail 42 (the far end of the tail from the head) and out of the sheath 33 at the outside surface of the head 43.

This internal duct 44 of the tip 41 comprises a portion oriented in the axial direction, narrowing from the proximal end of the tail 42, configured in order that the portion of the tube 11 of the straw 10 situated in the neighborhood of the end 17 (furthest end from the stopper 12) can enter the narrowing portion of the duct 44 and advance to a stop position in which the portion of the tube 11 situated in the neighborhood of the end 17 is clamped around by the wall of that portion of the duct 44.

This clamping round provides at the same time the stop for the pushing-in of the straw 10 into the sheath 33 and the liquid-tightness between the straw 10 and the sheath 33.

The rigid tubular body 39 of the gun 32, in which the straw 10 was placed in advance, is inserted into the sheath 33 by its open end with the straw 10 first, the straw inserts into the duct 44 of the tip 41. The insertion into the sheath 33 ends when the straw 10 comes to bear against the wall of the narrowing duct 44.

The sheath 33 is then fastened to the rigid tubular body 39 of the gun 32, in general in the neighborhood of the proximal end of the sheath 33 (open end of the tube 40) for example with a suitable ring.

The tube 11 of the straw 10 is thus immobilized relative to the assembly formed by the tubular body 39 of the gun 32 and by the sanitary sheath 33 fastened to that body, since the end 16 of the tube 11 bears against the shoulder of the tubular body 39 of the gun 32 and the end 17 bears against the wall of the narrowing portion of the duct 44.

The piston 46 can then be used to drive the rod and make the stopper 12 of the straw 10 slide so as to eject the semen out of the tube 11 and out of the tube 40 of the sheath 33 by the duct 44 which opens outside the sheath 33 at the outside surface of the head 43.

To perform the insemination, the apparatus 20 must of course have been inserted into the animal, and the work tool 19 mounted on the apparatus 20.

The apparatus 20 is inserted into the animal by the inseminator who can monitor the advancement of the speculum tube 23 on the remotely located screen.

It will be noted that the fact that the diameter of the outside surface 29 of the end part 26 increases from the opening 28 provides progressiveness which facilitates the insertion of the speculum tube 23.

Once the apparatus 20 is in place, that is to say when the opening 28 of the tube 23 faces opposite the uterine cervix, the work tool 19 is inserted through the handling shaft 21, the distal end 24 first, and advanced until the extension 45 is pushed into the handling shaft 21 as far as the first detent stop.

It is also possible to so insert the work tool 19 into the apparatus 20 before insertion of the apparatus 20 into the animal.

During such insertion of the tool 19 into the apparatus 20, the distal end 24 of the sheath 33 passes entirely through the handling shaft 21, then is inserted into the speculum tube 23 and passes through the guiding support 50.

The assembly 30 then attains the configuration illustrated in Figure 1, which is an initial configuration in which the work tool 19 is in a withdrawn position relative to the apparatus 20 and the piston 46 is out as far as possible from the extension 45 (the semen not yet having been ejected).

The extension 45 is then pushed forward, detent stop by detent stop, to make it slide within the handling shaft 21 until the work tool 19 comes into the advanced position.

During this movement, the distal end 24 of the tool 19 passes through the opening 28 of the tube 23 and advances within the vagina of the animal until it is inserted into the uterine cervix.

The inseminator can monitor the advancement of the distal end 24 on the remotely located screen and thus take care not to injure the animal.

The sliding detent stop by detent stop of the extension 45 relative to the handling shaft 21 also makes it possible to control the advancement of the distal end 24.

It will be noted that the objective 35 is maintained by the support 49 in a predetermined position such that the distal end 24 enters into the field of view of the objective 35 (and thus becomes visible on the remotely located screen) before having passed through the opening 28. Thus, so long as the inseminator cannot identify the distal end 24 on the screen, he can be sure that no part of the tool 19 protrudes from the opening 28 and projects into the vagina. As a matter of fact, such a part of the tool 19 protruding from the opening 28 would lead to a risk of injuring the animal at the time of possible uncontrolled movements of the apparatus 20.

It will furthermore be noted that since the work tool 19 is centered relative to the outside surface of the speculum tube 23, it is also centered relative to the vagina.

The assembly 30 then attains the configuration illustrated in Figure 4, which is an intermediate configuration in which the work tool 19 is in an advanced position and the piston 46 is still out as far as possible from the extension 45.

It then only remains for the inseminator to push the piston 46 to eject the semen and terminate the insemination.

For more details on the arrangement of the front part of the apparatus 20, at which are located the support block 47 and the end part 26 of the tube 23, please refer to international application WO 2019/122685, to which corresponds U.S. patent application US 2021/0085169.

### SUMMARY OF THE INVENTION

The invention is directed to provide an insemination work tool of the same kind but with improved performance in terms of convenience of use while being simple and economical.

The invention accordingly provides an insemination work tool comprising an insemination gun and a gun extension, said insemination gun comprising a rigid tubular body, a rod slidingly mounted in said rigid tubular body between an advanced position and a retracted position, a body head fastened to a proximal end of the rigid tubular body, a rod head fastened to a proximal end of the rod, the rod head being away from the body head in the retracted position whereas the rod head is by the body head in said advanced position, said rigid tubular body being configured for receiving in a predetermined position a straw filled with a predetermined dose of liquid-based substance, in particular pure or diluted animal semen, said rigid tubular body with said straw received therein in said predetermined position being configured for being inserted into a sanitary sheath configured to be fastened to said rigid tubular body so that a tube of said straw is immobilized relative to the assembly formed by said rigid tubular body and said sanitary sheath, said rod being configured for driving relative to said tube a stopper of said straw of which the tube is so immobilized so as to eject said predetermined dose of liquid-based substance out of said tube of said straw and out of a tube of said sanitary sheath by driving said rod from said retracted position to said advanced position, said rod coming to bear on said stopper which is then in an initial position relative to said tube of said straw and making the stopper slide in the manner of a piston up to a final position of the stopper which is by the end of the tube of the straw which is the furthest from the initial position of the stopper, said gun extension having an elongated shape with a mechanical fastener at a front end, said gun extension being configured for being fastened by said mechanical fastener to said rigid tubular body through said body head so as to take an operative position in which said gun extension is maintained aligned with the rigid tubular body, said gun extension surrounding in said operative position a space in which the rod head passes between the retracted position and the advanced position; characterized in that said gun extension has a rear transverse member and a longitudinal window between said rear transverse member and said mechanical fastener, said window having a length at least equal to the stroke of the rod head between the retracted position and the advanced position and a width enabling a finger of an adult to push the rod head from the retracted position to the advanced position.

Unlike the gun extension 45 of the prior art insemination work tool 19 described above, the gun extension of the insemination work tool according to the invention enables the inseminator, thanks to the longitudinal window between the rear transverse member and the mechanical fastener, to act on the rod of the insemination gun directly with a finger, without needing a further part such as the piston 46.

Still unlike the prior art insemination work tool 19, the insemination work tool according to the invention offers in the ready to use configuration a good protection against unexpected efforts oriented longitudinally and frontwards exerted on the rear of the insemination work tool, in particular thanks to the rear transverse member which prevents such efforts to be exerted on the rod, thus preventing losses of the high value substance packaged in the straw.

According to advantageous features of the insemination work tool according to the invention:
- said window has a length greater than the stroke of the rod head between the retracted position and the advanced position so as to enable said finger of an adult to insert between the rear transverse member and the rod head while the rod is in the retracted position;
- said gun extension has a floor facing a space between the rear transverse member and the rod head while the rod is in the retracted position;
- said gun extension comprises, further to said longitudinal window, hereinafter termed first longitudinal window, a second longitudinal window between said rear transverse member and said mechanical fastener, said second longitudinal window facing said first longitudinal window;
- said gun extension comprises a front transverse member comprising said mechanical fastener and further comprises a first lateral member and a second lateral member; said rear transverse member, said first lateral member, said front transverse member and said second lateral member being generally arranged as a rectangular frame;
- said gun extension comprises a front transverse member delimiting a snap-in recess for receiving said body head such that said front transverse member forms said mechanical fastener;
- said front transverse member comprises a first transverse wall and a second transverse wall, said snap-in recess comprising a pocket delimited frontwards by the first transverse wall and rearwards by the second transverse wall, said snap-in recess further comprising a front slot in the first transverse wall and a rear slot in the second transverse wall, the first transverse wall and the second transverse wall being configured for accommodating said body head in said pocket, accommodating said rod in said rear slot and accommodating said rigid tubular body in said front slot;
- said first transverse wall and said second transverse wall are configured for immobilizing longitudinally said body head in said pocket, for immobilizing radially said rigid tubular body in said front slot and for loosely accommodating said rod in said rear slot;
- said first transverse wall has a bottom bridge, a first flange and a second flange, said bottom bridge extending from the first lateral member to the second lateral member, the first flange being connected to the bottom bridge and to the first lateral member, the second flange being connected to the bottom bridge and to the second lateral member, said front slot being delimited by an edge face of the first flange and an edge face of the second flange, each said edge face having a first section extending between an end of said front slot up to a first cusp, a second section extending from said first cusp to a second cusp and a third section extending from the second cusp to the bottom bridge, the first section and the second section of the edge face of the first flange facing the first section and the second section of the edge face of the second flange, the third section of the edge face of the first flange and the third section of the edge face of the second flange facing the bottom bridge, each first section being slanted such that the first section of the edge face of the first flange gets closer to the first face of the edge face of the second flange towards the bottom bridge, each second section being concave with a curvature corresponding to the profile of the rigid tubular body, said front transverse member being configured such that upon insertion of said body head into said pocket said rigid tubular body contacts the first section of the edge face of the first flange and the second section of the edge face of the second flange and slides thereon driving them away from each other until the rigid tubular body passes over the first cusp of the edge face of the first flange and the first cup of the edge face of the second flange, the first flange and the second flange then getting closer while the second section of the edge face of first flange and the second section of the edge face of the second flange clamp therebetween the rigid tubular body;
- said rigid tubular body has a circular profile in a neighboring region to said body head, said body head is a circular collar extending radially from said neighboring region of said rigid tubular body, and said front transverse member is arranged such that when said body head is received in said pocket and said neighboring region of said rigid tubular body is clamped between the second section of the edge face of first flange and the second section of the edge face of the second flange said body head can be rotated as a wheel for rotating said rigid tubular body about its axial direction;

- said gun extension comprises along each lateral side of said longitudinal window a rim having at mid-length a cut-out configured for accommodating a finger of an adult;
- said gun extension comprises a first lateral member and a second lateral member each having a longitudinal groove;
- said gun extension comprises in a front region of each said longitudinal groove a resiliently retractable member of a detent pusher; and/or
- at least one of said first lateral member and second lateral member comprises a transverse stop wall at a rear end of said longitudinal groove.

The invention is also directed to a work assembly comprising an insemination work tool as disclosed above and an apparatus for assisting in vaginal penetration, provided to receive said insemination work tool, said apparatus comprising: a handle; a speculum tube extending from the handle to a distal end at which it has an opening, said handle having a handle rail configured to cooperate with said gun extension for slidingly guiding the insemination work tool in the speculum tube in a predetermined manner such that the insemination work tool can reach an advanced position in which part of the insemination work tool projects beyond the opening of the speculum tube; a video viewing system comprising an objective disposed within the speculum tube and a transmission device connected to the objective and connectable to a remotely located screen for viewing the image obtained by said objective; and an objective support to hold the objective in a predetermined position relative to the speculum tube in which the objective is in the neighborhood of said opening of the speculum tube and faces the space located beyond said opening of the speculum tube.

### BRIEF DESCRIPTION OF THE DRAWINGS

The description of the invention now continues with a detailed description of example embodiments given hereinafter by way of non-limiting examples with reference to the appended drawings. In these drawings:
- Figure 1, previously described, is a side view of a prior art work assembly comprising an apparatus for assisting in vaginal penetration and a work tool received in this apparatus, this work tool comprising an insemination gun and a gun extension, the prior art work assembly being in an initial configuration in which the work tool is in a withdrawn position on the handling shaft of the apparatus, in which the end of the work tool is located inside the speculum tube of the apparatus;
- Figure 2, previously described, is a diagrammatic view in longitudinal cross-section of a straw for the preservation of a predetermined dose of liquid-based substance, in particular pure or diluted animal semen, suitable for being used with the work tool;
- Figure 3, previously described, is a longitudinal section view of the end portion of the assembly which can be seen on the right in Figure 1, showing the work tool with the preservation straw of Figure 2 received inside, the video viewing system and a support block configured to slidingly guide the work tool and hold the objective of the video viewing system in a predetermined position;
- Figure 4, previously described, is a similar view to Figure 1 but showing the assembly in an intermediate configuration in which the work tool is in an advanced position on the handling shaft of the apparatus, in which the end of the work tool projects forward from the speculum tube;
- Figure 5, previously described, is a similar view to Figure 4 but showing the assembly in a final configuration in which the semen has been ejected out from the straw and from the work tool;

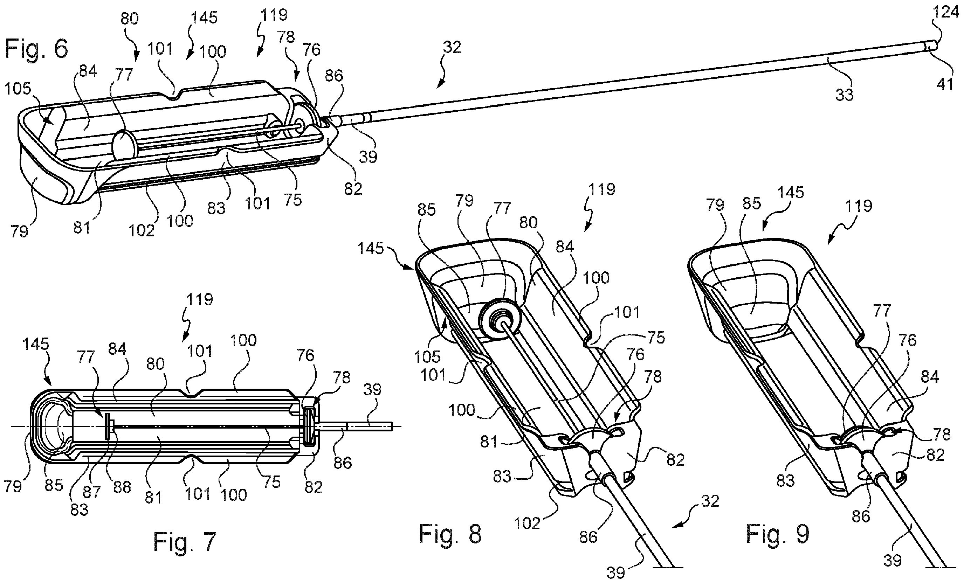

- Figure 6 is a perspective view of an insemination work tool according to the invention, comprising an insemination gun and a gun extension, the insemination gun being the same as in the work assembly shown in Figures 1 to 5, equipped with a same semen preservation straw and a same sanitary sheath, the rod of the insemination gun being in the retracted position, the gun extension being different from the gun extension of the work assembly shown in Figures 1 to 5;

- Figures 7 and 8 show the insemination work tool according to the invention as in Figure 6 but respectively in top view and in perspective view taken from an angle different than in Figure 6;
- Figure 9 is a view similar to Figure 8 but with the rod of the insemination gun in the advanced position;

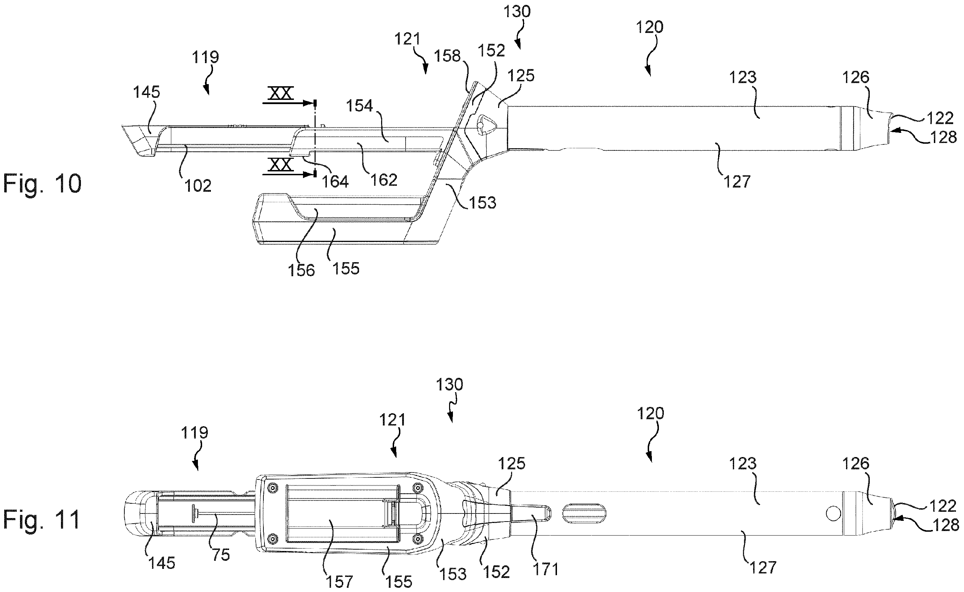

- Figure 10 is a side view of a work assembly according to the invention, comprising the insemination work tool shown on Figures 6 to 9 and an apparatus for assisting in vaginal penetration receiving this insemination work tool, the work assembly being, as the prior art work assembly shown in Figure 1, in an initial configuration in which the work tool is in a withdrawn position on the handle of the apparatus and in which the end of the work tool is located inside the speculum tube of the apparatus while the rod of the insemination gun is in the retracted position;
- Figures 11 to 13 show the work assembly according to the invention as in Figure 10, but respectively in bottom view and in partial perspective views taken from two different angles;

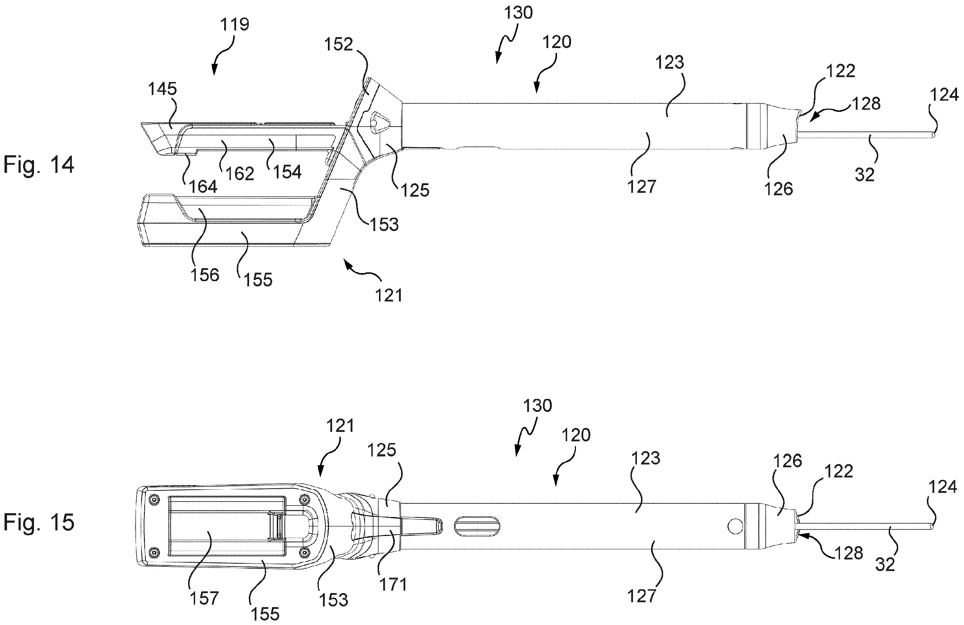

- Figures 14 to 17 are views similar to Figures 10 to 13, respectively, but with the work assembly, as the prior art work assembly shown in Figure 4, in an intermediate configuration in which the work tool is in an advanced position on the handle of the apparatus, in which the end of the work tool projects forward from the speculum tube;

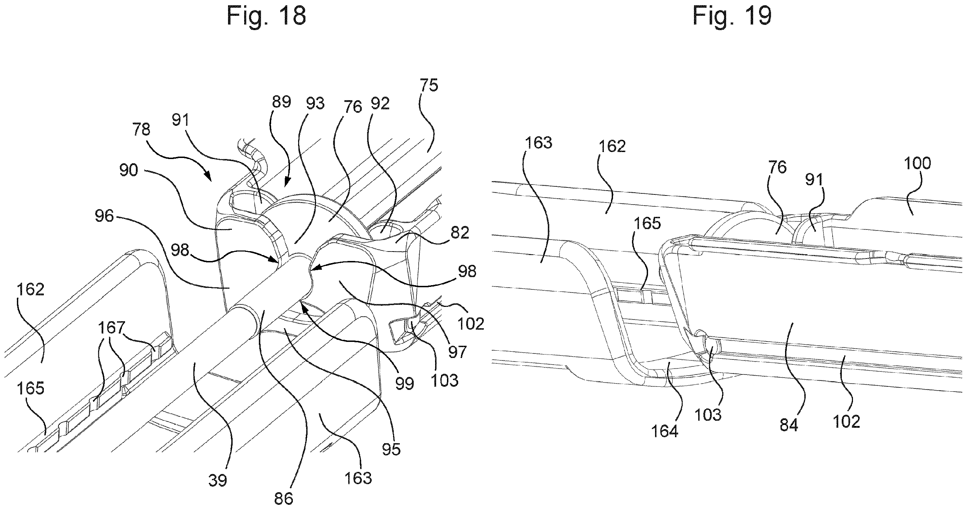

- Figure 18 is a partial perspective view showing the insemination work tool according to the invention and the handle guide of the apparatus of the assembly according to the invention in a configuration in which the gun extension is about to be received into the handle guide;
- Figure 19 show the insemination work tool and the work assembly as in Figure 18 but in a partial perspective view taken from a different angle;

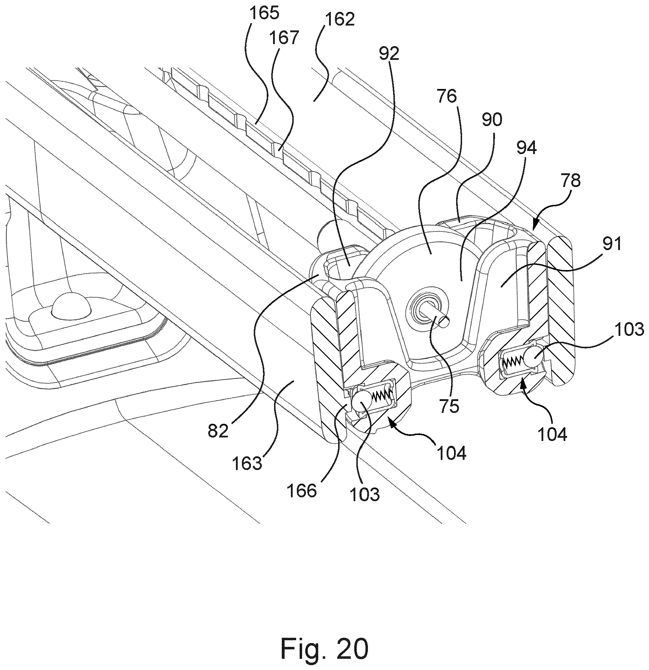

- Figure 20 is a partial perspective-section view, taken as shown by a cross-sectional plan "XX-XX" on Figure 10;

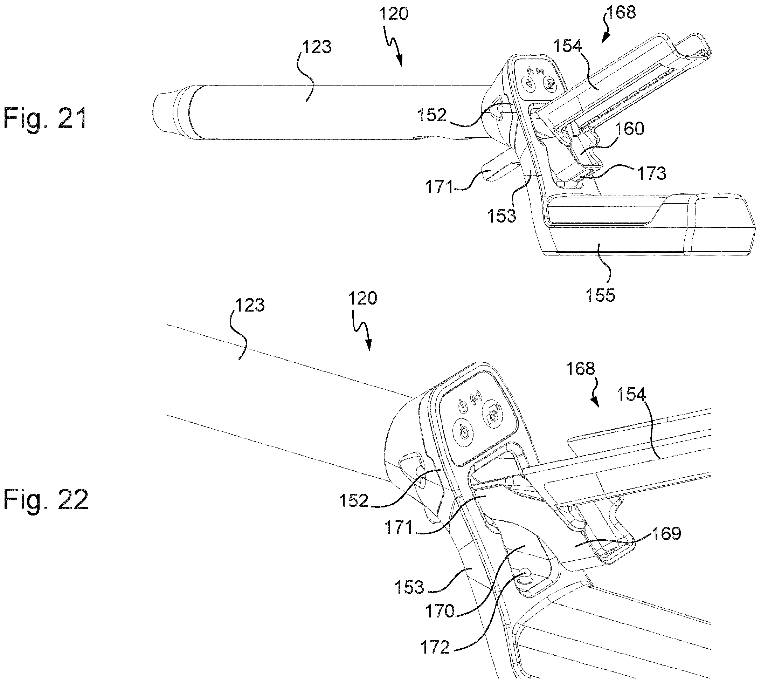

- Figure 21 is a perspective view of the apparatus of the work assembly according to the invention showing the handle guide in a position from which it can be removed from the rest of the apparatus; and
- Figure 22 is a partial perspective view showing the handle guide in the course of being removed from the rest of the apparatus.

### DETAILED DESCRIPTION OF EXAMPLE EMBODIMENTS

The insemination work tool 119 shown on Figures 6 to 9 comprises a re-usable conventional insemination gun 32 and a gun extension 145.

The re-usable conventional insemination gun 32 is equipped with a semen preservation straw 10 and a single-use sanitary sheath 33 as already described with reference to Figures 1 to 5.

Further to what is shown in Figures 1 to 5, it will be noted that the conventional insemination gun 32 comprises a body head 76 fastened to the proximal end of the rigid tubular body 39 as well as a rod head 77 fastened to the proximal end of the rod 75.

The rod 75 is slidingly mounted in the rigid tubular body 39 between an advanced position shown on Figures 6 to 8 and a retracted position shown on Figure 9.

The rod head 77 is away from the body head 76 in the retracted position whereas the rod head 77 is by the body head 76 in the advanced position.

In Figures 6 to 8, the re-usable conventional insemination gun 32 is shown ready to use, i.e. equipped with a semen preservation straw 10 and a sanitary sheath 33 while the rod 75 is in the retracted position.

Prior to insertion of the straw 10 into the rigid tubular body 39, the inseminator here takes directly the rod 75, through the rod head 77, as far as possible out from the rigid tubular body 39, so as to place the rod 75 in the retracted position shown on Figures 6 to 8.

For making the stopper 12 of the straw 10 slide so as to eject the semen out of the tube 11 and out of the tube 40 of the sheath 33, the inseminator here acts directly on the rod 75, through the rod head 77, by driving the rod 75 from the retracted position (Figures 6 to 8) to the advanced position (Figure 9), the rod 75 coming to bear on the stopper 12 which is then relative to tube 11 in the initial position shown on Figure 2, and making the stopper 12 slide in the manner of a piston up to a final position of the stopper 12 which is by the end 17 of the tube 11 of the straw 10 which is the furthest from the initial position of the stopper 12.

The gun extension 145 has an elongated shape with a mechanical fastener 78 at a front end.

The gun extension 145 is configured for being fastened by the mechanical fastener 78 to the rigid tubular body 39 through the body head 76 so as to take the operative position illustrated on Figures 6 to 20.

In this operative position, the gun extension 145 is maintained aligned with the rigid tubular body 39.

The arrangement of the gun extension 145 is such that in the operative position the gun extension 145 surrounds a space in which the rod head 77 passes between the retracted position (Figures 6 to 8) and the advanced position (Figure 9).

The gun extension 145 has a rear transverse member 79 and a longitudinal window 80 between the rear transverse member 79 and the mechanical fastener 78.

The window 80 has a length longer than the stroke of the rod head 77 between the retracted position (Figures 6 to 8) and the advanced position (Figure 9) and a width enabling a finger of an adult to insert between the rear transverse member 79 and the rod head 77 while the rod 75 is in the retracted position and then to push the rod head 77 from the retracted position (Figures 6 to 8) to the advanced position (Figure 9).

For instance, the stroke of the stopper 12 between the initial position and the final position may be of the order of 116 mm, and the stroke of the rod 75 between the retracted position and the advanced position may be of the order of 125 mm.

For instance, the window 80 may have a width of the order of 20 mm and a length of the order of 147 mm.

It will be noted that in the prior art gun extension 45 shown on Figures 1, 4 and 5 the only openings are at the front end and at the rear end, the opening at the front end being surrounded by the peripheral mechanical fastener, the opening at the rear end enabling to receive the piston 46.

Unlike the prior art gun extension 45, the gun extension 145 enables the inseminator, thanks to the window 80, to act on the rod 75 directly with a finger, without needing a further part such as the piston 46.

It will further be noted that in the prior art insemination work tool 19, in the ready to use configuration shown on Figure 1, an unexpected effort oriented longitudinally and frontwards exerted on the rear of the insemination work tool 19 might inadvertently drive the piston 46 inside the gun extension 45 and cause losses of the high value substance packaged in the straw 10.

Unlike the prior art insemination work tool 19, the insemination work tool 119 in the ready to use configuration shown on Figures 6 to 8 offers a good protection against unexpected efforts oriented longitudinally and frontwards exerted on the rear of the insemination work tool 119, in particular thanks to the rear transverse member 79 which prevents such efforts to be exerted on the rod 75, thus preventing losses of the high value substance packaged in the straw 10.

Here, the gun extension 145 comprises, further to the longitudinal window 80, a longitudinal window 81 between the rear transverse member 79 and the mechanical fastener 78, the longitudinal window 81 facing the longitudinal window 80.

For instance, the window 81 may have a width of the order of 21 mm and a length of the order of 133 mm.

The window 81 enables the inseminator to grasp the rod head 77 between two fingers, such as the thumb and the index, one finger reaching the rod head 77 through the window 80 and the other finger reaching the rod head 77 through the window 81.

The gun extension 145 will now be described in more details.

Here, further to the rear transverse member 79 the gun extension 145 comprises a front transverse member 82 comprising the mechanical fastener 78 and further comprises a first lateral member 83 and a second lateral member 84.

The rear transverse member 79, the first lateral member 83, the front transverse member 82 and the second lateral member 84 are generally arranged as a rectangular frame.

Here, the gun extension 145 further comprises a floor 85 (Figures 7 to 9) facing a space between the rear transverse member 79 and the rod head 77 when the rod 75 is in the retracted position (Figures 6 to 8).

The floor 85 can form a stop for a finger entering via the window 80 the space that the floor 85 faces, thus helping to position the finger at a depth within the gun extension 145 which is suitable for pushing the rod head 77 from the retracted position to the advanced position.

The floor 85 is also useful for rigidifying the gun extension 145.

The front transverse member 82 will now be described in details.

Before starting that description, it should be noted that here the rigid tubular body 39 comprises on a certain length from the body head 76 a section 86 of greater external diameter and that here the body head 76 is a circular collar extending radially from the neighbouring region of circular profile formed by the section 86.

It should also be noted that the rod head 77 comprises here a circular collar 87 and a boss 88 (figure 7) between the rod 75 and the collar 87. Here the collar 87 and the boss 88 are concentric to the rod 75.

For forming the mechanical fastener 78, the front transverse member 82 delimits a snap-in recess 89 (Figure 18) for receiving the body head 76.

As seen on Figures 18 to 20, the front transverse member 82 comprises a first transverse wall 90 and a second transverse wall 91.

The snap-in recess 89 comprises a pocket 92, a front slot 93 and a rear slot 94 (Figure 20).

The pocket 92 is delimited frontwards by the first transverse wall 90 and rearwards by the second transverse wall 91. The front slot 93 is in the first transverse wall 90. The rear slot 94 is in the second transverse wall 91.

The first transverse wall 90 and the second transverse wall 91 are configured for accommodating the body head 76 in the pocket 92, accommodating the rod 75 in the rear slot 94 and accommodating the rigid tubular body 39 in the front slot 93.

More precisely, here the first transverse wall 90 and the second transverse wall 91 are configured for immobilizing longitudinally the body head 76 in the pocket 92, for immobilizing radially the rigid tubular body 39 in the front slot 93 and for loosely accommodating the rod 75 in the rear slot 94.

The first transverse wall 90 has a bottom bridge 95, a first flange 96 and a second flange 97. The bottom bridge 95 extends from the first lateral member 83 to the second lateral member 84. The first flange 96 is connected to the bottom bridge 95 and to the first lateral member 83. The second flange 97 is connected to the bottom bridge 95 and to the second lateral member 84.

The front slot 93 is delimited by an edge face of the first flange 96 and an edge face of the second flange 97. Each of these edge faces has a first section extending between an end of the front slot 93 up to a first cusp 98, a second section extending from the first cusp 98 to a second cusp 99 and a third section extending from the second cusp 99 to the bottom bridge 95. The first section and the second section of the edge face of the first flange 96 faces the first section and the second section of the edge face of the second flange 97. The third section of the edge face of the first flange 96 and the third section of the edge face of the second flange 97 face the bottom bridge 95.

Here, the edge face of the second flange 97 is arranged as the mirror image of the edge face of the first flange 96.

The first section of the edge face of the first flange 96 and the first section of the edge face of the second flange 97 are each slanted such that the first section of the edge face of the first flange 96 gets closer to the first face of the edge face of the second flange 97 towards the bottom bridge 95.

The second section of the edge face of the first flange 96 and the second section of the edge face of the second flange 97 are each concave with a curvature corresponding to the profile of the rigid tubular body 39, and more precisely here to the profile of the neighbouring region to the body head 76 formed by the section 86 of greater external diameter.

The front transverse member 82 is configured such that during insertion of the body head 76 into the pocket 92 the rigid tubular body 39, and more precisely here the neighbouring region to the body head 76, contacts the first section of the edge face of the first flange 96 and the second section of the edge face of the second flange 97 and slides thereon driving them away from each other until the rigid tubular body 39 passes over the first cusp 98 of the edge face of the first flange 96 and the first cup 98 of the edge face of the second flange 97, the first flange 96 and the second flange 97 then getting closer while the second section of the edge face of first flange 96 and the second section of the edge face of the second flange 97 clamp therebetween the rigid tubular body 39, and more precisely here the neighbouring region to the body head 76.

It should be noted that the slant orientation of the first section of the edge face of the first flange 96 and of the first section of the edge face of the second flange 97 is such that the effort exerted thereon by the rigid tubular body 39 during the insertion of the body head 76 into the pocket 92 causes the first flange 96 and the second flange 97 to move apart from each other thanks to an elastic bending of the bottom bridge 95. When the first cusps 98 are passed over, the bottom bridge 95 tends to elastically return to its initial configuration so that the first flange 96 and the second flange 97 are driven towards each other and clamp the rigid tubular body. The second cusp 99 is located on the edge face of the first flange 96 and of on the edge face of the second flange 97 such that an effort exerted on the body head 76 while the rigid tubular body is clamped as just described between the second sections of the edge faces causes the first flange 96 and the second flange 97 to get closer to one another so that the rigid tubular body do not passes over the second cusps 99.

The front transverse member 82 is arranged such that when the body head 76, in the form of a circular collar, is received in the pocket 92 and the rigid tubular body 39, and more precisely here its section 86 neighbor to the body head 76, is clamped between the second section of the edge face of first flange 96 and the second section of the edge face of the second flange 97, the body head 76 can be rotated as a wheel for rotating the rigid tubular body 39 about its axial direction.

Indeed, the second section of the edge face of first flange 96 and the second section of the edge face of the second flange 97 play the role of a bearing for the rigid tubular body 39.

Such possibility of rotating the rigid tubular body 39 can be of help in certain circumstances during insertion of the distal end of the insemination work tool 19 into the uterine cervix of the animal.

The gun extension 145 comprises along each lateral side of the longitudinal window 80 a rim 100 having at mid-length a cut-out 101 configured for accommodating a finger of an adult.

Here, the rims 100 are each formed by a curved end portion of a wall of the first lateral member 83 or of the second lateral member 84.

The possibility of accommodating a finger in a cut-out 101 can help the user to push the rod 75, for instance by putting the index of one hand into the cut-out 101 and using the thumb of this hand for pushing the rod head 77 by bringing the thumb closer to the index. The rod head 77 can be so driven for about half its stroke. Then the user can put for example its index against the external surface of the front transverse member 82 and push the rod head 77 with its thumb by bringing the thumb closer to the index, so as to drive the rod head 77 for the remainder of its stroke.

Here, the slot 94 (Figure 20) is large enough for accommodating loosely the rod 75 and also the boss 88 (Figure 7) of the rod head 77 so that the boss 88 can contact the body head 76.

Here, as more particularly visible on Figures 6 and 19, the first lateral member 83 and the second lateral member 84 each has a longitudinal groove 102.

Here, as more particularly visible on Figures 18 to 20, the gun extension 145 comprises in a front region of each longitudinal groove 102 a resiliently retractable member 103 of a detent pusher 104 (Figure 20).

Here, the resiliently retractable member 103 is a ball.

Here, the first lateral member 83 and the second lateral member 84 each comprises a transverse stop wall 105 (Figures 6 and 8) at a rear end of the longitudinal groove 102.

The transverse stop wall 105 is formed here by a shoulder of a wall of the first lateral member 83 or of the second lateral member 84.

In a variant not illustrated, only one of the first lateral member 83 and the second lateral member 84 comprises a transverse stop wall 105.

Except the detent pusher 104, the gun extension 145 is here made in one and a single piece, for instance by injection molding of a plastic material such as PC/ABS, that is to say a mixture of PC (Polycarbonate) and ABS (Acrylonitrile Butadiene Styrene).

The insemination work tool 119 can be used alone for inseminating an animal.

The insemination work tool 119 can also be used in combination with the apparatus 120 for assisting in vaginal penetration, as shown on Figures 10 to 20.

Generally speaking, the apparatus 120 is similar to the apparatus 20 except that the handling shaft 21 is replaced by a handle 121 arranged differently.

For convenience, in the present memorandum the same reference numerals to which 100 is added are used for elements of the apparatus 120 similar to the elements of the apparatus 20.

The speculum tube 123 and the elements within the speculum tube 123 are generally arranged as the speculum tube 23 and the elements within the speculum tube 123.

The speculum tube 123 is fastened to the handle 121 via a fastening ring 125.

The ring 125 is fastened in the neighborhood of the proximal end of the speculum tube 123 (the end oriented towards the handle 121), here by bonding.

The fastening ring 125 is attached to the distal end of the handle 121 (the end oriented towards the speculum tube 123), here by snap engagement.

The ring 125 and the handle 121 are configured so that the ring 125 can have its snap engagement with the handle 121 undone, the speculum tube 123 thus being removable.

The speculum tube 123 comprises an end part 126 and a main part 127. The end part delimits the opening 128 at the distal end 122 of the speculum tube 123. The main part 127 extends between the end part 126 and the handle 121.

In other words, the end part 126 extends between the main part 127 and the distal end 122 of the speculum tube 123 which is also the distal end of the end part 126.

The opening 128 is delimited by the edge of the end part 126 located at the distal end 122.

The end part 126 is of elastically deformable material, whereas the main part 127 is of rigid material.

Generally speaking, the description of the end part 26 and of the main part 27 given above applies to the end part 126 and to the main part 127.

The elements within the speculum tube 123 are the same as the elements within the speculum tube 23, in particular a video viewing system comprising an objective and a transmission device connected to the objective and connectable to a remotely located screen for viewing the images obtained by the objective; as well as support block and a mounting bar.

Generally speaking, the description given above of the video viewing system of the apparatus 20 applies to the video viewing system of the apparatus 120, except that there is no plug socket 37, the energy required for operation of the video viewing system coming from a battery 157 (Figures 11 and 15) housed in the handle 121 whereas the communication with the remotely located screen is wireless, here via Wi-Fi.

Generally speaking, the description given above of the support block 47 and the mounting bar 48 of the apparatus 20 applies to the support block and the mounting bar of the apparatus 120, except that the guiding support of the apparatus 120 is configured in order for the work tool 119 to slidingly move in an axial direction which is not centered relative to the outside surface of the speculum tube 123 but close to the wall of the end part 126 away from the objective support.

For instance, the working tool 119 may be positioned at a distance of the order of 7 mm from a central axis relative to the outside surface of the speculum tube 123.

As visible in Figure 14, the location of the portion of the insemination work tool 119 projects beyond the distal end 122 of the speculum tube 123.

As explained below, the gun extension 145 is configured, in particular thanks to the longitudinal grooves 102, to be slidingly mounted in the handle 121 such that the portion of the work tool 119 that is received in the speculum tube 123 is able to slide through the guiding support of the apparatus 120 (which is similar to the guiding support 50 of the apparatus 20).

The insemination work tool 119 is thus mounted movably in terms of translation relative to the apparatus 120 between a position that is withdrawn (Figures 10 to 13) relative to the apparatus 120, in which the distal end 124 of the work tool 119 is located inside the speculum tube 123 (that is to say inside the internal space of the tube 123 delimited by its wall), and a position that is advanced relative to the apparatus 120 (Figures 14 to 17), in which the distal end 124 of the insemination tool 119 projects forwardly of the speculum tube 123.

As mentioned above, the apparatus 120 is generally similar to the apparatus 20 except that the handling shaft 21 is replaced by a handle 121 arranged differently.

The handle 121 comprises a handle core 152 from which the speculum tube 123 extends, a handle leg 153 extending from the handle core 152 at an angle to the speculum tube 123, and a handle rail 154 extending from the handle core 152 away from and aligned with the speculum tube 123.

The handle core 152 has a passage for the insemination work tool 119 between the handle rail 154 and the speculum tube 123.

The handle rail 154 is configured to slidingly guide the work tool 119 in the speculum tube 123 in the manner described above.

Here, the handle core 152 comprises a top face defining a flat top edge of the handle 121.

The handle core 152 is configured such that the apparatus 120 or the work assembly 130 can be stably positioned upside down on a horizontal surface, such as the top of a table, the apparatus 120 or the work assembly 130 resting by the flat top edge of the handle core 152 and by the gun extension 145 or the handle rail 154 on the horizontal surface, such that the speculum tube 123 is not oriented towards the horizontal surface.

Thanks to such positioning, the apparatus 120 or the work assembly 130 prevents the speculum tube 123 from getting contaminated by the horizontal surface, or conversely prevents the horizontal surface from getting contaminated by the speculum tube 123.

Here, the handle 121 further comprises a handle foot 155 connected to the handle leg 153 at the opposite end to the handle core 152.

The handle foot 155 is oriented as the speculum tube 123 and the handle rail 154, i.e. the general direction of the handle foot 155 is parallel to the general direction of the speculum tube 123 and to the general direction of the handle rail 154.

The handle foot 155 faces the handle rail 154.

The handle foot 155 includes a battery housing 156 configured for accommodating a removable battery 157 (Figures 11 and 15) from which comes the energy required for operation of the video viewing system.

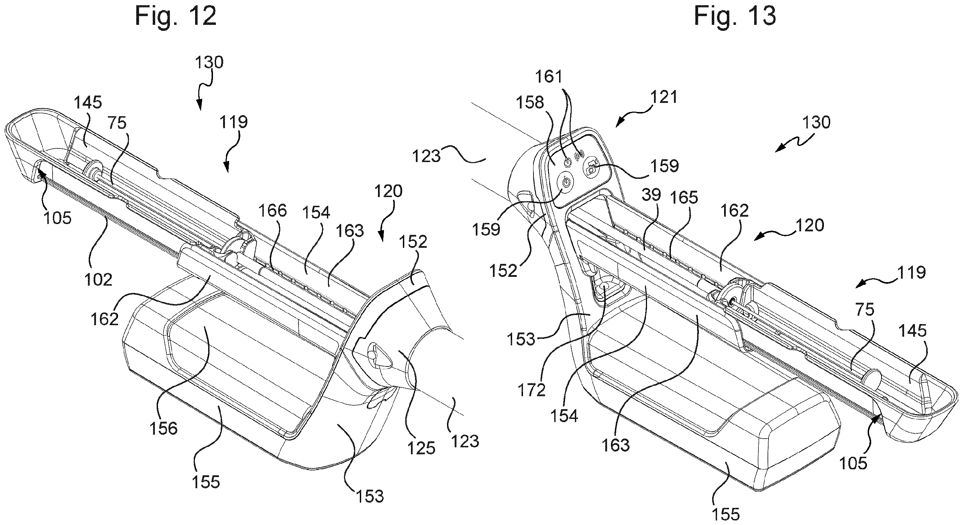

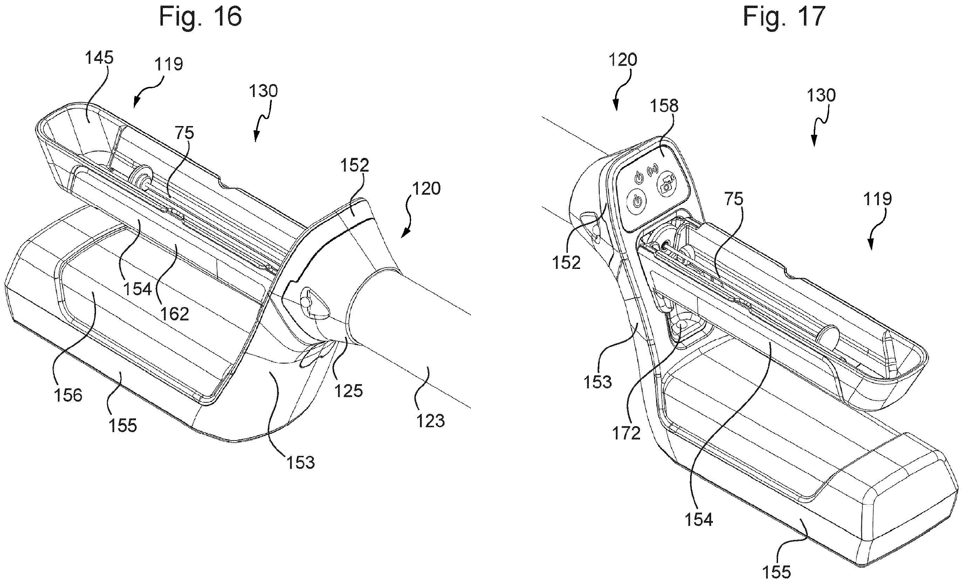

The handle core 152 has above the passage for the insemination work tool 119 a panel 158 (Figures 13 and 17) on which is arranged a control interface having at least one button 159, here two buttons 159 and two indicators 161.

Here, there is an on/off button, a video or photo recording button, an on/off indicator and a wireless communication indicator.

The handle rail 154 here has a first longitudinal wall 162, a second longitudinal wall 163, a floor 164 (Figures 10, 14 and 19), a first longitudinal rib 165 and a second longitudinal rib 166.

Here, the first longitudinal wall 162, the second longitudinal wall 163 and the floor 164 are arranged so that the handle rail 154 has a U profile, with the first longitudinal wall 162 and the second longitudinal wall 163 corresponding to the lateral branches of the U and the floor 164 corresponding to the central branch of the U.

Here, the floor 164 is located only at the rear of the handle rail 154. The bottom of the handle rail 154 is thus open between the floor 164 and the handle core 162.

The first longitudinal rib 165 projects internally from the first longitudinal wall 162. The second longitudinal rib 166 projects internally from the second longitudinal wall 163. The first longitudinal rib 165 and the second longitudinal rib 166 face each other and are parallel to each other.

Here, the first longitudinal rib 165 and the second longitudinal rib 166 are located respectively near the bottom of the first longitudinal wall 162 and near the bottom of the second longitudinal wall 163.

As mentioned above, the handle rail 154 is configured to slidingly guide the work tool 119.

More precisely, the handle rail 154 is configured to slidingly guide the gun extension 145.

As seen on Figures 18 to 20, the gun extension 145 fits in the handle rail 154 with the first longitudinal rib 165 received in the longitudinal groove 102 of the first lateral member 83 and the second longitudinal rib 166 received in the longitudinal groove 102 of the second lateral member 84.

Here, the first longitudinal rib 165 and the second longitudinal rib 166 each comprises notches 167 regularly spaced therealong with a predetermined pitch.

The notches 167 are configured for cooperating with the resiliently retractable member 103 so as to form detent stops for positioning the work tool 119 relative to the apparatus 120. The predetermined pitch is approximately 1 cm here.

To perform the insemination, the apparatus 120 must of course have been inserted into the animal, and the work tool 119 mounted on the apparatus 120.

The apparatus 120 is inserted into the animal by the inseminator who can monitor the advancement of the speculum tube 123 on the remotely located screen.

Once the apparatus 120 is in place, that is to say when the opening 128 of the tube 123 faces opposite the uterine cervix, the work tool 119 is inserted through the handle 121, the distal end 124 first, and advanced as shown of Figures 18 to 20 until the extension 145 is pushed into the handle rail 154 as far as the first detent stop.

It is also possible to so insert the work tool 119 into the apparatus 120 before insertion of the apparatus 120 into the animal.

During such insertion of the tool 119 into the apparatus 120, the distal end 124 passes through the handle 121, then is inserted into the speculum tube 123 and passes through the guiding support (which is similar to the guiding support 50 of the apparatus 20).

The work assembly 130 then attains the configuration illustrated in Figures 10 to 13, which is an initial configuration in which the work tool 119 is in a withdrawn position relative to the apparatus 120 and the rod 75 is out as far as possible from the rigid tubular body 39 (the semen not yet having been ejected).

The extension 145 is then pushed forward, detent stop by detent stop, to make it slide within the handle rail 154 until the work tool 119 comes into the advanced position.

During this movement, the distal end 124 of the tool 119 passes through the opening 128 of the tube 123 and advances within the vagina of the animal until it is inserted into the uterine cervix.

The inseminator can monitor the advancement of the distal end 124 on the remotely located screen and thus take care not to injure the animal.

The sliding detent stop by detent stop of the extension 145 relative to the handle rail 154 also makes it possible to control the advancement of the distal end 124.

The assembly 130 then attains the configuration illustrated on Figures 14 to 17, which is an intermediate configuration in which the work tool 119 is in an advanced position and the rod 75 is still out as far as possible from the rigid tubular body 39.

It then only remains for the inseminator to push the rod 75 to eject the semen and terminate the insemination.

It should be noted that the rims 100 of the extension 145 are over the longitudinal walls 162 and 163 and that in the advanced position (Figures 14 to 17) the cut-outs 101 remain accessible so that they can accommodate a finger of the inseminator for helping him to push the rod 75 as described above.

It should be noted that the open nature of the bottom of the handle rail 154 between the floor 164 and the handle core 162 allow the inseminator to grasp the rod head 77 between two fingers as described above. The same applies to the body head 76.

It should be noted that in the advanced position of the work tool 119 (Figures 14 to 17) the transverse stop wall 105 formed by a shoulder of a wall of the first lateral member 83 or of the second lateral member 84 is against the rear edge face of the handle rail 154. The transverse stop wall 105 thus forms an end stop for the stroke of the gun extension 145.

As visible on Figures 21 and 22, the handle rail 154 makes part of a handle guide 168 removably mounted in the apparatus 120.

Further to the handle rail 154, the handle guide 168 comprises a tenon 169 to be received in a mortise 170 provided in the handle core 152 and the handle leg 153.

The handle guide 168 also comprises a nose 171 to be received in a notch of the handle core 152 and a notch of the speculum tube 123, as visible on Figures 11 and 15.

When the handle guide 168 is mounted in the rest of the apparatus 120, the nose 171 helps guiding the work tool 119.

The tenon 169 is maintained in the mortise 170 by snap-in engagement, here thanks to a resiliently retractable member 172, here a finger of a detent pusher.

For removing the handle guide 168, the user pushes onto the retractable member 172. This frees the handle guide 168, which can be taken out for instance by first inclining the handle rail 154 as shown on Figure 21, by raising it rear upwards, and then by moving the handle rail 154 rearwards as shown on Figure 22.

The handle guide 168 can be mounted on the rest of the apparatus 120 with movements reverse to those just described. Thanks to a groove 173 (Figure 21) ending in a hole for receiving the retractable member 172, the handle guide 168 can be snapped-in into the remainder of the apparatus 120.

It should be noted that the removable nature of the handle guide 168 enables to use the remainder of the apparatus 120 for purposes different than carrying out an artificial insemination with an insemination gun 32, for instance operating the remainder of the apparatus 120 for imaging purposes only or with a different handle guide for a different work tool, for instance a tool for depositing drugs, a tool for listening to sounds or a tool for sampling internal substances.

It should be noted that since the speculum tube 123 and also the handle guide 168 are removable, the apparatus 120 is very easy to clean since after removal the speculum tube 123 and the handle guide 168 can be immersed in a cleaning medium while the remainder of the apparatus 120, which is minimized, can be easily cleaned by wiping.

In variants not shown:
- the window 80 has a length smaller but at least equal to the stroke of the rod head 77;
- there is no rear floor as 85 and/or there is a floor having a greater length;
- the sanitary sheath has no tip and instead has a folded rim forming a hem at its distal end and a sliding sleeve disposed inside the sheath, the sleeve being configured to receive an end of the straw and provide sealing between the sheath and the straw when the straw pushes the sleeve to come to bear against the hem;

- the video viewing system only comprises a single lighting member juxtaposed against the objective;
- the photosensor is not of CCD type but is for example of CMOS type;
- the transmission device of the video viewing system has no electronic circuit, no photosensor and no cable but instead comprises an optic fiber cable;
- the transmission device has a plug socket to connect thereto a remotely located screen and/or comprises a wireless connection device different than Wi-Fi, for instance Bluetooth;
- the transmission device comprises a projector configured to project the image onto a medium forming the remotely located screen, the medium for example being a wall or a piece of fabric;
- the offset screen is not that of a smartphone but that of a tablet, a PC, glasses and/or of a projector;
- the fastening ring for fastening the speculum tube to the handling shaft has no snap-engagement members and instead comprises bayonet type fastening members;
- the material of the speculum tube is different from a thermoplastic material and is for example of glass;
- the speculum tube is not transparent but translucent or opaque;
- the end part of the speculum tube has a permanent mechanical connection to its main part, for example by being bonded;
- the end part of the speculum tube is not fitted by insertion into its main part but is butt-fitted thereto;
- the end part of the speculum tube overlaps its main part on the outside;
- the speculum tube has no main part and end part, but a single continuous part as described in PCT application WO 2016/066962, to which corresponds U.S. patent application US 2017/0319317.

Numerous other variants are possible according to circumstances, and in this connection it is to be noted that the invention is not limited to the examples described and shown.

## Claims

1. Insemination work tool comprising an insemination gun (32) and a gun extension, said insemination gun (32) comprising a rigid tubular body (39), a rod (75) slidingly mounted in said rigid tubular body (39) between an advanced position and a retracted position, a body head (76) fastened to a proximal end of the rigid tubular body (39), a rod head (77) fastened to a proximal end of the rod (75), the rod head (77) being away from the body head (76) in the retracted position whereas the rod head (77) is by the body head (76) in said advanced position, said rigid tubular body (39) being configured for receiving in a predetermined position a straw (10) filled with a predetermined dose of liquid-based substance, in particular pure or diluted animal semen, said rigid tubular body (39) with said straw (10) received therein in said predetermined position being configured for being inserted into a sanitary sheath (33) configured to be fastened to said rigid tubular body (39) so that a tube (11) of said straw (10) is immobilized relative to the assembly formed by said rigid tubular body (39) and said sanitary sheath (33), said rod (75) being configured for driving relative to said tube (11) a stopper (12) of said straw of which the tube is so immobilized so as to eject said predetermined dose of liquid-based substance out of said tube (11) of said straw (10) and out of a tube (40) of said sanitary sheath (33) by driving said rod (75) from said retracted position to said advanced position, said rod (75) coming to bear on said stopper (12) which is then in an initial position relative to said tube (11) of said straw (10) and making the stopper (12) slide in the manner of a piston up to a final position of the stopper (12) which is by the end (17) of the tube (11) of the straw (12) which is the furthest from the initial position of the stopper (12), said gun extension having an elongated shape with a mechanical fastener at a front end, said gun extension being configured for being fastened by said mechanical fastener to said rigid tubular body (39) through said body head (76) so as to take an operative position in which said gun extension is maintained aligned with the rigid tubular body, said gun extension surrounding in said operative position a space in which the rod head (77) passes between the retracted position and the advanced position; **characterized in that** said gun extension (145) has a rear transverse member (79) and a longitudinal window (80) between said rear transverse member (79) and said mechanical fastener (78), said window (80) having a length at least equal to the stroke of the rod head (77) between the retracted position and the advanced position and a width enabling a finger of an adult to push the rod head (77) from the retracted position to the advanced position.

2. Insemination work tool according to claim 1, wherein said window (80) has a length greater than the stroke of the rod head (77) between the retracted position and the advanced position so as to enable said finger of an adult to insert between the rear transverse member (79) and the rod head (77) while the rod (75) is in the retracted position.

3. Insemination work tool according to claim 2, wherein said gun extension (145) has a floor (85) facing a space between the rear transverse member (79) and the rod head (77) while the rod (75) is in the retracted position.

4. Insemination work tool according to any one of claims 1 to 3, wherein said gun extension (145) comprises, further to said longitudinal window (80), hereinafter termed first longitudinal window, a second longitudinal window (81) between said rear transverse member (79) and said mechanical fastener (78), said second longitudinal window (81) facing said first longitudinal window (80).

5. Insemination work tool according to any one of claims 1 to 4, wherein said gun extension (145) comprises a front transverse member (82) comprising said mechanical fastener (78) and further comprises a first lateral member (83) and a second lateral member (84); said rear transverse member (79), said first lateral member (83), said front transverse member (82) and said second lateral member (84) being generally arranged as a rectangular frame.

6. Insemination work tool according to any one of claims 1 to 5, wherein said gun extension (145) comprises a front transverse member (82) delimiting a snap-in recess (89) for receiving said body head (76) such that said front transverse member (82) forms said mechanical fastener (78).

7. Insemination work tool according to claim 6, wherein said front transverse member (82) comprises a first transverse wall (90) and a second transverse wall (91), said snap-in recess (89) comprising a pocket (92) delimited frontwards by the first transverse wall (90) and rearwards by the second transverse wall (91), said snap-in recess (89) further comprising a front slot (93) in the first transverse wall (90) and a rear slot (94) in the second transverse wall (91), the first transverse wall (90) and the second transverse wall (91) being configured for accommodating said body head (76) in said pocket (92), accommodating said rod (75) in said rear slot (94) and accommodating said rigid tubular body (39) in said front slot (93).

8. Insemination work tool according to claim 7, wherein said first transverse wall (90) and said second transverse wall (91) are configured for immobilizing longitudinally said body head (76) in said pocket (92), for immobilizing radially said rigid tubular body (39) in said front slot (93) and for loosely accommodating said rod (75) in said rear slot (94).

9. Insemination work tool according to claim 8, wherein said first transverse wall (90) has a bottom bridge (95), a first flange (96) and a second flange (97), said bottom bridge (95) extending from the first lateral member (83) to the second lateral member (84), the first flange (96) being connected to the bottom bridge (95) and to the first lateral member (83), the second flange (97) being connected to the bottom bridge (95) and to the second lateral member (84), said front slot (93) being delimited by an edge face of the first flange (96) and an edge face of the second flange (97), each said edge face having a first section extending between an end of said front slot (93) up to a first cusp (98), a second section extending from said first cusp (98) to a second cusp (99) and a third section extending from the second cusp (99) to the bottom bridge (95), the first section and the second section of the edge face of the first flange (96) facing the first section and the second section of the edge face of the second flange (97), the third section of the edge face of the first flange (96) and the third section of the edge face of the second flange (97) facing the bottom bridge (95), each first section being slanted such that the first section of the edge face of the first flange (96) gets closer to the first face of the edge face of the second flange (97) towards the bottom bridge, each second section being concave with a curvature corresponding to the profile of the rigid tubular body (39), said front transverse member (82) being configured such that upon insertion of said body head (76) into said pocket (92) said rigid tubular body (39) contacts the first section of the edge face of the first flange (96) and the second section of the edge face of the second flange (97) and slides thereon driving them away from each other until the rigid tubular body (39) passes over the first cusp (98) of the edge face of the first flange (96) and the first cup (98) of the edge face of the second flange (97), the first flange (96) and the second flange (97) then getting closer while the second section of the edge face of first flange (96) and the second section of the edge face of the second flange (97) clamp therebetween the rigid tubular body (39).

10. Insemination work tool according to claim 9, wherein said rigid tubular body (39) has a circular profile in a neighboring region (86) to said body head (76), said body head (76) is a circular collar extending radially from said neighboring region (86) of said rigid tubular body (39), and said front transverse member (82) is arranged such that when said body head (76) is received in said pocket (92) and said neighboring region (86) of said rigid tubular body (39) is clamped between the second section of the edge face of first flange (96) and the second section of the edge face of the second flange (97), said body head can be rotated as a wheel for rotating said rigid tubular body (39) about its axial direction.

11. Insemination work tool according to any one of claims 1 to 10, wherein said gun extension (145) comprises along each lateral side of said longitudinal window (80) a rim (100) having at mid-length a cut-out (101) configured for accommodating a finger of an adult.

12. Insemination work tool according to any one of claims 1 to 11, wherein said gun extension (145) comprises a first lateral member (83) and a second lateral member (84) each having a longitudinal groove (102).

13. Insemination work tool according to claim 12, wherein said gun extension (145) comprises in a front region of each said longitudinal groove (102) a resiliently retractable member (103) of a detent pusher (104).

14. Insemination work tool according to claim 12 or claim 13, wherein at least one of said first lateral member (83) and second lateral member (84) comprises a transverse stop wall (105) at a rear end of said longitudinal groove (102).

15. Work assembly comprising an insemination work tool (119) according to any one of claims 1 to 14 and an apparatus (120) for assisting in vaginal penetration, provided to receive said insemination work tool (119), said apparatus (120) comprising:
- a handle;
- a speculum tube (123) extending from the handle to a distal end (122) at which it has an opening (128), said handle having a handle rail (154) configured to cooperate with said gun extension (145) for slidingly guiding the insemination work tool (119) in the speculum tube (123) in a predetermined manner such that the insemination work tool (119) can reach an advanced position in which part of the insemination work tool (119) projects beyond the opening (128) of the speculum tube (123);
- a video viewing system comprising an objective disposed within the speculum tube (123) and a transmission device connected to the objective and connectable to a remotely located screen for viewing the image obtained by said objective; and
- an objective support to hold the objective in a predetermined position relative to the speculum tube (123) in which the objective is in the neighborhood of said opening (128) of the speculum tube (123) and faces the space located beyond said opening (128) of the speculum tube (123).
